# EUROPEAN PATENT APPLICATION

(11) **EP 2 335 730 A1**
(43) Date of publication of application: **22.06.2011**
(21) Application number: 09015725.6
(22) Date of filing: 18.12.2009
(51) Int. Cl.: A61K 39/395, C12N 15/62

(54) **Fusion polypeptides and their use for prevention and treatment of cancer**

(71) Applicant: Deutsches Krebsforschungszentrum, 69120 Heidelberg (DE)
(72) Inventor: Cid-Arregui, Angel, 69126 Heidelberg (DE); zur Hausen, Harald, 69483 Waldmichelbach (DE)
(74) Representative: Schüssler, Andrea

(57) **Abstract**

Described are immunogenic polypeptides and genes useful as vaccines for prevention and treatment of cancer. The vaccine contains a recombinant fusion polypeptide comprising - in any order - (a) at least one immune stimulatory polypeptide, (b) at least one transmembrane (TM) polypeptide, and (c) at least one tumor antigen, generating humoral and cellular responses against tumor cells. Also described is a nucleic acid sequence encoding this fusion polypeptide as well as vectors, preferably viral vectors, containing this nucleic acid sequence. Preferably, this fusion polypeptide comprises MIP-3β, HBsAg(S), IL-2, E6 and E6, and, optionally a His-Tag.

## Description

The present invention provides immunogenic polypeptides and genes for prevention and treatment of cancer. The invention provides recombinant fusion polypeptides comprising - in any order - (a) at least one immune-stimulatory polypeptide, (b) at least one transmembrane (TM) polypeptide, and (c) at least one tumor antigen, generating humoral and cellular immune responses against tumor cells. The present invention also provides nucleic acid sequences encoding such fusion polypeptides as well as vectors, preferably viral vectors, containing these nucleic acid sequences. Preferably, said nucleic acid sequences are codon-optimized for translation in mammals, yeast, or other organisms.

Despite enormous investments of financial and human resources, cancer remains one of the major causes of death. Standard accepted approaches for treatment of cancers such as leukaemias, solid tumors, and metastases include surgery, radiotherapy, and chemotherapy. It is believed, however, that the human immune system is a potential resource to eradicate cancer cells and that effective treatment can be developed if the components of the immune system are properly stimulated to recognize and eliminate cancer cells. Thus, immunotherapy, which comprises compositions and methods to activate the body's immune system, either directly or indirectly, to shrink or eradicate cancer, has been studied for many years as an adjunct to conventional cancer therapy.

It is generally admitted that the growth and metastasis of tumors depends largely on their capacity to evade host immune surveillance. Most tumors express antigens that can be recognized to a variable extent by the host immune system, but in many cases, the immune response is inadequate. Failure to elicit a strong activation of effector T-cells may result from the weak immunogenicity of tumor antigens or inappropriate or absent expression of co-stimulatory molecules by tumor cells. For most T-cells, production of IL-2 and proliferation require a co-stimulatory signal simultaneous with TCR engagement, otherwise, T-cells may enter a functionally unresponsive state, known as clonal anergy.

Immunotherapy of cancer has been described in the art for a number of approaches, including early attempts of cell-based cancer vaccines consisting of killed autologous tumor cells or tumor cell lysates mixed with adjuvants, such as *Bacillus* Calmette Guerin (BCG) and *Corynebacterium parvum,* in an attempt to amplify tumor-specific immune responses (Berd et al., J. Clin. Oncol. 8: 1858-1867 [1990]). However, due to the weak immunogenicity of many cancers, down regulation of MHC molecules, the lack of adequate costimulatory molecules and secretion of immuno-inhibitory cytokines by cancer cells, the response to such vaccines has not resulted in long term efficacy. (Bodey et al., Anticancer Res [2000] 20:2665-2676; Armstrong and Jaffee, Surg Oncol Clin N Am. [2002] 11:681-696). Currently, the most popular genetically modified cell-based vaccines take advantage of the large set of cloned genes encoding cytokines and co-stimulatory molecules (Pardoll, Ann. Rev. Immunol [1995] 13: 399-415; Pardol, Nat Med. [2007] 13:1411-1413).

Subsequently, genetically modified tumor vaccines replaced the most complex and inconsistent mixtures of tumor cells and bacteria, in particular active vaccination of patients with tumor associated antigens (TAAs) and tumor-specific antigens (TSAs) with the aim to raise an immune response in these individuals that recognizes and destroys the cancer cells.

During the years, many cancer antigens have been described for this purpose, including:
(a) antigens expressed selectively by cancer cells and cells of the male germinal line, such as the MAGE family antigens (MAGE, BAGE, GAGE, RAGE, NY-ESO-1);
(b) proteins encoded by differentiation genes activated in cancer cells (for instance, tyrosinase, gp100, MelanA, TRP-1 and TRP-2);
(c) proteins that represent neoantigens as consequence of gene mutations frequently occurring in cancer cells (Pardoll, Annu. Rev. Immunol. [2003] 21:807-839), e.g. mutated CDK4, catenin B, caspase 8, *ras, p53;*
(d) proteins overexpressed in tumor cells, such as *p53, Her-2*/*neu,* PRAME, the prostatic acid phosphatase, MUC1, *p16^{ink4a};* and
(e) viral antigens like the E6 and E7 proteins of the so-called high-risk human papillomaviruses (HPVs), which cause pre-cancer lesions and cancers of the anogenital epithelium and head and neck tumors, and the EBNA1 product of the Epstein-Barr virus (EBV).

The concept of adoptive cellular therapy for tumors, first presented nearly 50 years ago by Mitchison (Mitchison, N. A.: J Exp Med. (1955) 102: 157-77), has at its goal the elimination of cancer through the transfer of activated T-cells and/or natural killer cells. Adoptive immunotherapy is based on the belief that tumor specific cytotoxic T-cells are present in cancer patients, but that such cells have not been primed and/or that the *in vivo* function of the cells is impaired. To prime the cells, peripheral T-cells are removed from the patient, activated *ex vivo,* and then re-infused. The step of *ex vivo* activation also may include exposure to the patient's tumor cells or to a tumor cell vaccine. Although T-cell based adoptive immunotherapy provides a potentially promising form of cancer treatment, it has failed to induce a long-lasting response in the majority of patients who have received this therapy (Lum et al., J Immunother. (2001) 24: 408-19).

Further immunotherapeutic strategies include gene therapy, administration of small molecular inhibitors and activators, anti-sense oligonucleotides, vaccines, activated autologous cells, antibodies, as well as cytokines and chemokines, natural or recombinant proteins, autologous cells modified in vitro (Mocellin et al., Cancer Immunol. & Immunother. [2002] 51: 583-595; Dy et al., J. Clin. Oncol. [2002] 20: 2881-2894).

In spite of the length of time that these therapies have been investigated, there remains a need for improved strategies for enhancing the immune response against the tumor antigens. Such strategies include the combination of the tumor antigens with powerful vaccine adjuvants and immune stimulatory molecules. Numerous cytokines have been shown to play a role in regulation of the immune response to tumors. For example, U.S. Patent No. 5,098,702 describes using combinations of TNF, IL-2 and IFN-β in synergistically effective amounts to combat existing tumors. U.S. Patent Nos. 5,078,996, 5,637,483 and 5,904,920 describe the use of GM-CSF for treatment of tumors. However, direct administration of cytokines for cancer therapy may not be practical, as they are often systemically toxic; see, for example, Asher et al., J. Immunol. 146: 3227-3234, 1991 and Havell et al., J. Exp. Med. 167: 1067-1085, 1988).

Nevertheless, there is a need in the art for safe and effective compositions that can stimulate the immune system as a cancer immunotherapy.

According to the invention, safe and effective stimulation of the immune system as a cancer immunotherapy is achieved by the subject matters defined in the claims. The present invention is based on the applicant's findings that the immune response against tumor cells can be enhanced by application of complex polypeptides (or synthetic genes encoding them) comprising fusions of a carrier polypeptide, tumor-derived antigenic polypeptides and immunostimulatory molecules. Such complex polypeptides can be used, as single molecular species or as mixtures of several of them, in prophylactic or therapeutic settings, also in combination with current conventional therapies. Thus, the present invention relates to novel vaccine formulations based on composite fusion proteins comprising at least the following components: (i) a carrier polypeptide molecule able to self-assemble into nanoparticles, preferably virus-like particles (VLPs), (ii) one or more tumor antigen sequences or immunologically relevant epitopes of these, (iii) one or more immunostimulatory domains, and, optionally, (iv) one or more encapsulated, conjugated or associated components, which enhance the immunogenicity of the complex. In preferred embodiments, the vaccines of the invention are used in the treatment of cancer of the uterine cervix and its precursor lesions (cervical intraepithelial neoplasia), pancreas carcinoma, hepatoma, non-small cell lung carcinoma (NSCLC), or bladder cancer. The vaccine of the present invention combines immunomodulators with tumor antigens (preferably, in virus-like particles) and is capable of evoking a stronger involvement of professional antigen presenting cells compared to the strategies used so far resulting in enhancement of tumor antigen-specific cytotoxic T cell lymphocytes.

### Brief description of the drawings

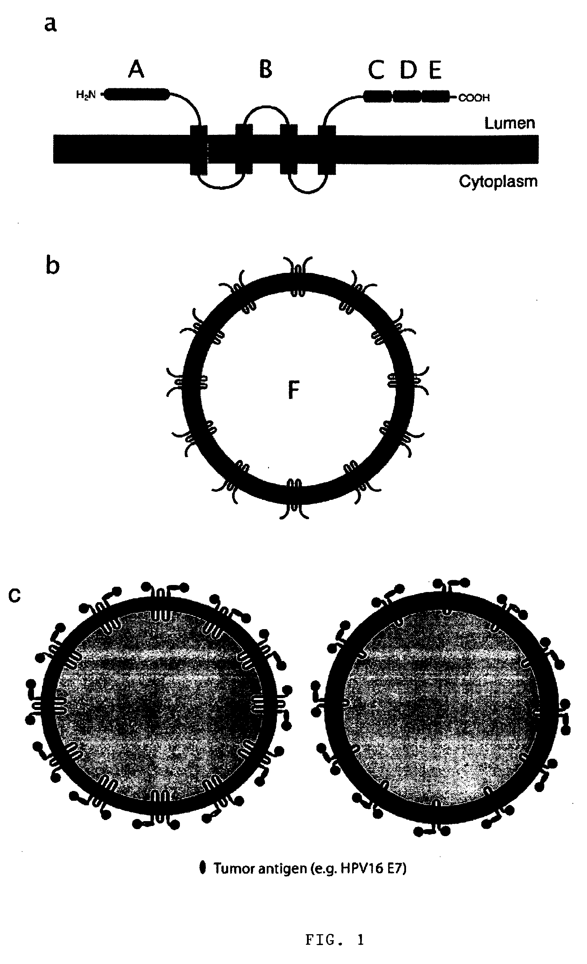

FIGURE 1: Schematic representation of the generic fusion polypeptide of the present invention and the particles formed in the Golgi compartment upon self-assembly of monomers
   **(A)** Diagram representing the fusion polypeptide as it is postulated to appear in the Golgi membranes. The different parts of the construct are labeled consecutively with letters A to E. The polyprotein comprises a core domain (B) made of an HBsAg(S) polypeptide, which contains four hydrophobic regions spanning the membrane. In this schematic depiction of the HBsAg(S) protein the N- and C-termini and the second hydrophilic region, which bears the major B-cell antigenic determinants (the "a" determinant region, amino acid residues 124-147), are external. Based on the Stirk model for the small S protein, HBsAg(S), the four transmembrane helices that are located between amino acid residues 8-28 (domain I), 79-100 (domain II), 160-184 (domain III) and 189-210 (domain IV) (Stirk et al, Intervirology. 1992;33:148-58). Domains A, C and D of the fusion polypeptide represent, interchangeably, two immunostimulatory domains and a tumor antigenic region. The domain E represents a Tag sequence (e.g., a His-Tag), which may serve for protein purification. Linker sequences separating each domain are not represented.
   **(B)** Schematic representation of a particle made of fusion protein monomers. Component F comprises compounds that enhance the antigenicity of the vaccine, such as antigenic peptides from the target tumor antigen.
   **(C)** Representation of particles made of fusion proteins containing the HBsAg(S) subunit (left side) or a deletion mutant (HBsAgΔ) (right side).
FIGURE 2: Sequence of a synthetic gene encoding the fusion polypeptide MHIH and the corresponding amino acid sequence
   The sequence was optimized for expression in human and mouse cells by converting its codons into those preferred in highly expressed human genes. Nucleotide sequence and derived amino acid sequence.
FIGURE 2(a): Sequence of a synthetic gene encoding the fusion polypeptide MHΔIH, carrying the deletion mutant HBsAgΔ¹⁵³ (having an amino-terminal deletion of the first 153 amino acids of the HBsAg(S) protein of 226 amino acids), and the corresponding amino acid sequence
   The sequence was optimized for expression in human and mouse cells by converting its codons into those preferred in highly expressed human genes.
FIGURE 3: Sequence of a synthetic gene encoding the fusion polypeptide MHIH optimized for expression in the yeast S. cerevisiae by converting its codons into those preferred in highly expressed yeast genes
FIGURE 3(a): Sequence of a synthetic gene encoding the fusion polypeptide MHΔIH, carrying the deletion mutant HBsAgΔ¹⁵³ (having an amino-terminal deletion of the first 153 amino acids of the HBsAg(S) protein of 226 amino acids), and the corresponding amino acid sequence
   The sequence was optimized for expression in human and mouse cells by converting its codons into those preferred in highly expressed yeast genes.
FIGURE 4: Expression and purification of MHIH particles with the EasySelect™ Picchia Yeast Expression System (Invitrogen)
   **(a)** Schematic representation of the plasmid pPICZ containing the AOX (alcohol oxygenase) expression cassette into which the gene MHIH was cloned under control of the AOX1 promoter, which is inducible by methanol. Upon transformation the expression cassette is incorporated into the AOX genomic sequence by homologous recombination.
   **(b)** Selection of *P. pastoris* transformants in conditioned medium containing escalating doses of the antibiotic Zeocin allows for selection of yeast clones containing high copy numbers of integrated expression cassette.
   (c) The integrity and copy number can be estimated by Southern blot analysis against known amounts of radiolabeled probe.

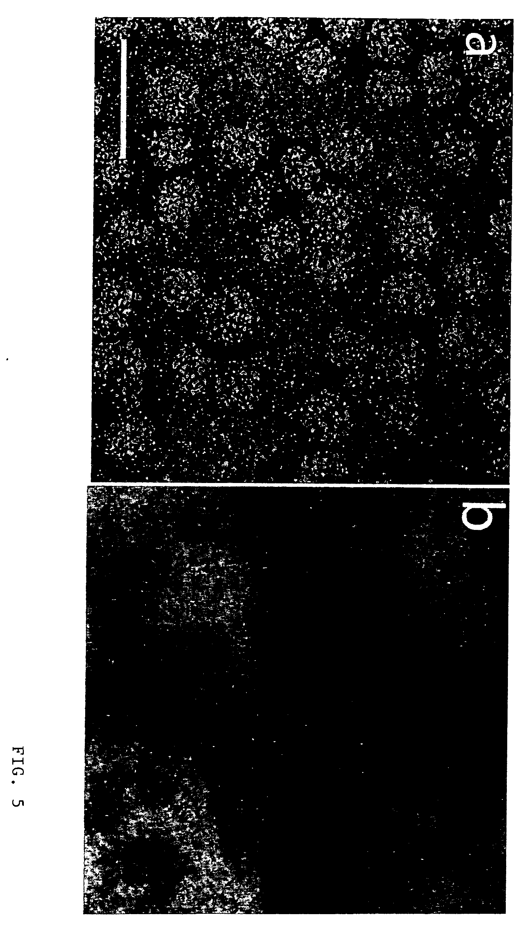

FIGURE 5: Transmission electron microscopy of **(a)** wild-type HBsAg(S) particles, and **(b)** particles made of the fusion polypeptide MHIH
   The particles were purified from yeast.
   Bar: 110 nm.

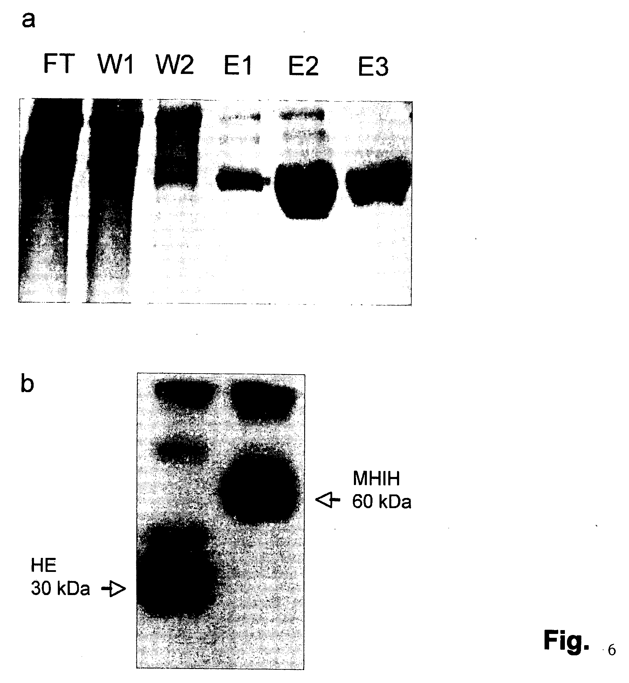

FIGURE 6: PAGE analysis of MHIH protein fractions purified from yeast by ion metal affinity chromatography
   **(a)** 10 µl of each fraction were loaded on a 12% polyacryl amide gel. After electrophoretic separation, proteins were stained with Coomassie blue. Fractions: Flow-through (FT), two consecutive wash fractions (W1 and W2) and three elution fractions (E1-E3) are shown. Typically, the highest amount of protein was obtained in fraction E2.
   **(b)** Western blot using, anti-MIP-3β antibodies. The HBs-E7 and MHIH are detected as bands of about 30 and 60 kDa, respectively.

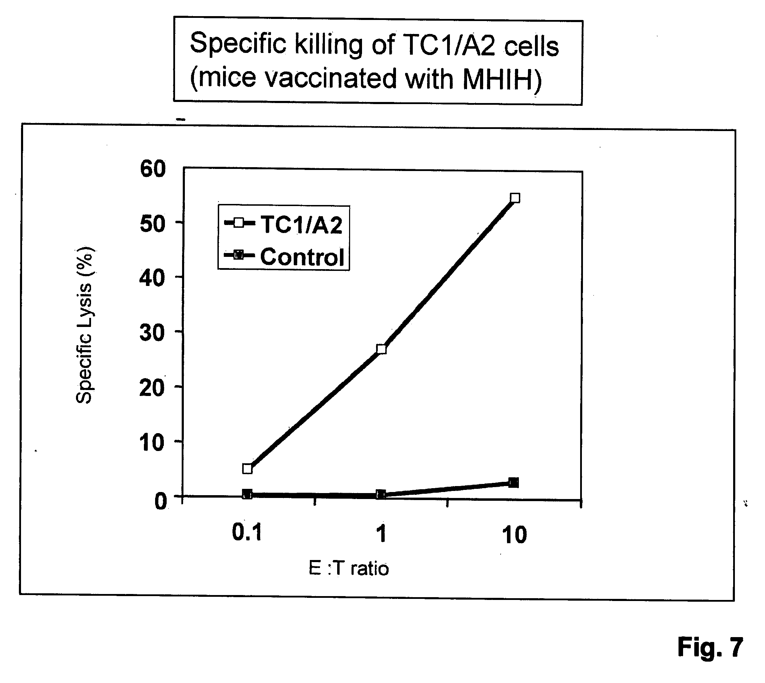

FIGURE 7: Cytotoxicity assay
   Specific killing of TC1/A2 cells by T cells from mice vaccinated with MHIH polypeptide-expressing plasmid. TC1/A2 cells express the E6 and E7 proteins of HPV16.

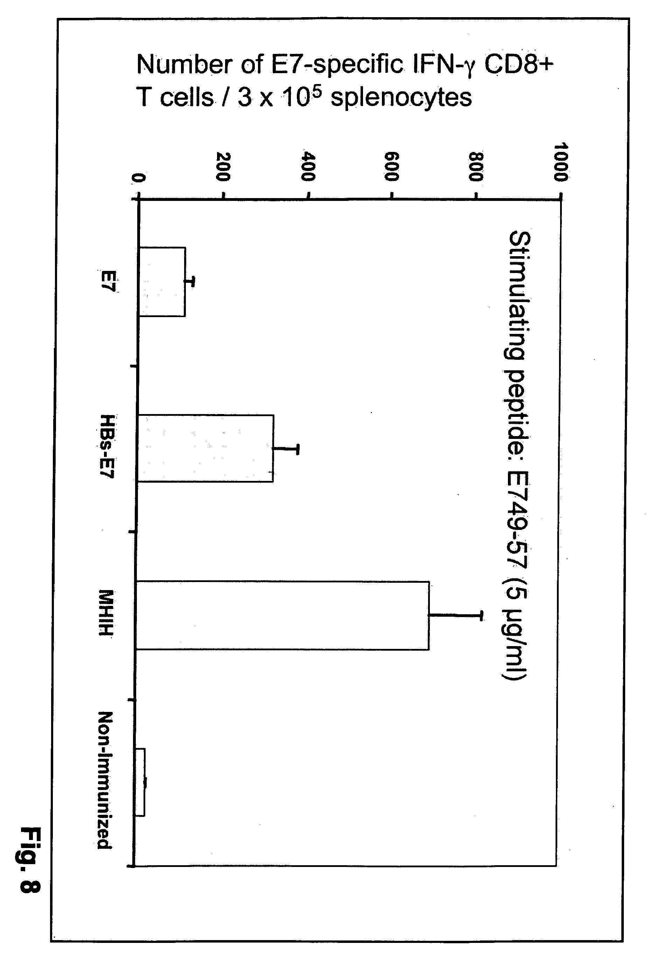

FIGURE 8: Comparison of E7 peptide-specific T cell numbers in splenocyte populations of mice immunized with E7, HBs-E7 and MHIH

The present invention provides a vaccine system based on nanoparticles (VLPs) suitable for targeting specific cells of the innate and adaptive immune system, wherein the nanoparticles comprise self-assembling protein domains that are optionally in the form of chimeric fusion proteins. In some embodiments, aspects of the invention relate to chimeric fusion proteins having the formula: N-terminus-AbCdE-C-terminus. In particular aspects of the invention, A and E are immunostimulatory and antigenic (e.g. tumor antigens) domains, C is a domain favoring self assembly, and b and d are linker peptides. In particular aspects of the invention, the domain favoring self-assembly is of viral origin and the targeting domains are of either viral or non-viral origin. In particular aspects of the invention, the targeting and antigenic domains of non-viral origin are of cellular origin, such as from a human or animal cell. In particular aspects of the invention, the linker may be of any natural or non-natural origin, including comprising synthetic de novo amino acids not found in nature. It should be appreciated that the chimeric fusion proteins may comprise fewer domains than AbCdE and any combination thereof, provided the chimeric fusion protein comprises at least the domain favoring self assembly (C). Examples for alternative chimeric fusion proteins are AbC, CdE, C, AC, CE, ACE, bCd, bC, or Cd. It also should be appreciated that in some embodiments one or more immunostimulatory and antigenic domains may be included in the C domain (e.g., as an insertion into the C domain sequence or as a replacement of part of the C domain sequence). It should be appreciated that the internal immunostimulatory domain(s) may be linked via a synthetic linker at either or both of the N-terminal and C-terminal ends of the targeting domain(s). However, the internal immunostimulatory and antigenic domains may be inserted without using a synthetic linker. It should be appreciated that a synthetic linker peptide as used herein is a peptide having a sequence that is different (or in a different location) from the natural sequence of A, C, or E, or a portion thereof.

The invention is based, at least in part, on the finding that certain modified forms of Hepatitis B Surface Antigen (HBsAg) can form nanoparticles that have improved properties for drug loading and delivery. In view of the prior knowledge in the art this finding was unexpected. In certain embodiments, the viral polypeptide sequence is derived from the surface antigen of the hepatitis B virus lacking one or more N-terminal transmembrane domains (e.g., a modified form of HBsAg comprising the amino acid sequence any of SEQ ID NO: 3). Accordingly, examples of such modified forms of HBsAg are HBsAgΔ98 (having an amino-terminal deletion of the first 98 amino acids of the HBsAg (S) protein of 226 amino acids) or HBsAgΔ153 (having an amino-terminal deletion of the first 153 amino acids of the HBsAg (S) protein of 226 amino acids). In some aspects, such modified forms of HBsAg retain the ability to self-assemble and to form nanoparticles. In some aspects, such modified forms of HBsAg may be expressed in and purified from yeast (for example, *P. pastoris*). In some aspects, such modified forms of HBsAg may form nanoparticles within a size range between, for example, 20 and 30 nm.

Nanoparticles can be formed using HBsAg protein variants that lack (for example, due to deletion and/or amino acid substitution) one or more N-terminal transmembrane domains of the S domain or chimeric fusion proteins thereof. Nanoparticles may be formed using variants of the S domain alone or in combination (for example, as a fusion protein or a mixture of proteins) with other HBsAg domains or other peptides or proteins. In certain embodiments, the chimeric fusion proteins include different moieties derived from various biological molecules.

Accordingly, aspects of the invention relate to nanoparticles having properties that promote efficient delivery of antigens to target tissues and cells. In some embodiments, nanoparticles based on modified HBsAg proteins of the invention are smaller than naturally-occurring hepatitis B viral particles and also smaller than particles based on HBsAg proteins that include the N-terminal transmembrane domains of the HBsAg S domain. In certain embodiments, HBsAg nanoparticles of the invention are smaller than 80 nm in diameter. Some HBsAg nanoparticles are smaller than 50 nm in diameter. Some HBsAg nanoparticles range between 10 and 40 nm in diameter. Some HBsAg nanoparticles range between 15 and 30 nm, and some may be about 20 nm to 30 nm in diameter. Certain nanoparticles of the invention are sufficiently small (for example, approximately 20 nm to 30 nm in diameter) to promote highly efficient cell entry (see, for example, Gao H. et al., PNAS 102(27):9469-74, 2005). However, it should be appreciated that larger nanoparticles of the invention (e.g., between 30 nm and 80 nm in diameter as described herein, or larger) also may be used to deliver antigens or other agents to target cells or tissues as described herein, as aspects of the invention are not limited in this respect.

Certain nanoparticles of the invention have a reduced density to optimize delivery capabilities. Without wishing to be bound by theory, some nanoparticles are expected to have less structural rigidity than their natural counterparts, thereby facilitating substance loading, delivery and/or escape from previous immune reactivity against wild-type HBsAg due to vaccination against hepatitis B or to infection with this the hepatitis B virus. For example, structural rigidity might be lowered in some nanoparticles as a result of fewer intra- and/or inter-molecular interactions due to the full or partial deletion and or substitution of one or more transmembrane domains of the HBsAg protein S domain. Without wishing to be bound by theory, this may lead to more flexible HBsAg S domains incorporated into the nanoparticle and/or to incorporation of more additional membrane material (such as for example lipids) into the nanoparticle. According to aspects of the invention, increased flexibility allows for improved immunogenicity of the antigenic domain and/or targeting properties of nanoparticles of the invention. However, nanoparticles of the invention may retain a uniform structure that is useful for fabrication and industrial scalability.

In some embodiments, nanoparticles of the invention are engineered to evade the immune system of a host (for example, a human subject, for example a subject that has been vaccinated against Hepatitis B) by removing (by deletion and/or substitution) antigenic amino acids or sequences in a membrane protein of the nanoparticle (for example, in the 'a' determinant region of the HBsAg protein). In certain embodiments, the non-cellular or viral sequence within the fusion protein is designed to be as small as possible to limit immunogenicity of this domain, which might hinder the immune responses against the antigenic domain by the subject.

Targeting molecules may be antibodies, ligands, receptors, or other molecules that can concentrate the nanoparticles at a target site, for example, by binding to a molecule that is preferentially expressed at the target cells, for example, natural killer (NK) cells, NKT cells, tumor-infiltrating lymphocytes or other cancer-specific immune cells or other target cells of interest for cancer immunotherapy).

In some embodiments, a nanoparticle of the invention (for example, lacking one or two transmembrane domains and/or an 'a' region of the HBsAg protein) may be used in a topical and/or parenteral administration.

Thus, the present invention provides a fusion polypeptide comprising in any order:
(1) at least one immune stimulatory polypeptide;
(2) at least one transmembrane (TM) polypeptide; and
(3) at least one tumor antigen; or
(4) a biologically active derivative or fragment of (1), (2), and/or (3).

As used herein the term "tumor" includes cancer cells, necrosis, as well as stroma. Stroma includes cells such as fibroblasts and endothelial cells of vessels and capillaries and extracellular matrix, which is composed of fibrillar and non-fibrillar components. The major fibrillar proteins are collagen and elastin. A cancer vaccine may target to the tumor by binding to the stroma which surrounds the cancer cells in the tumor. Thus, a cancer vaccine may target in the vicinity of a cancer by binding to a stromal component such as a fibroblast or endothelial cell or a component of the extracellular matrix.

Cancers treatable using the vaccine of the invention include carcinomas, sarcomas, leukaemias, lymphomas, and other types of cancer. Carcinomas include those of lung, breast, skin, colon, liver, pancreas, kidney, uterus, ovarian, prostate, and the like. These cancers may be primary or metastatic. In the case of leukaemias and lymphomas, the cancer cells treatable with the vaccine of the invention include those in the form of a tumor as well as cancer cells in the bone marrow and in the circulation.

The term "tumor antigen" as used herein comprises TAA and TSA. A "tumor associated antigen" (TAA) as used herein refers to a protein which is present on tumor cells, and on normal cells during fetal life (onco-fetal antigens), after birth in selected organs, or on normal cells, but at much lower concentration than on tumor cells. A TAA also may be present in the stroma in the vicinity of the cancer cell but be expressed at lower amounts in the stroma elsewhere in the body. A variety of TAAs have been described including mucins such as MUC1, BRCA-1 and BRCA-2 proteins, HER2/neu, integrins, cytokines, and the like. In contrast, the term "tumor specific antigen" (TSA) refers to a protein expressed by tumor cells, but absent from normal cells. TSAs usually appear when an infecting virus has caused the cell to become immortal and to express virus antigens. Exemplary viral TSAs are the E6 or E7 proteins of oncogenic HPV types, such as HPV-16 and HPV-18. TSAs not encoded by viruses can be idiotypes of the immunoglobulin on B cell lymphomas or the T cell receptor (TCR) on T cell lymphomas.

Tumor antigens can be a protein or peptide. "Polypeptide", "peptide" and "protein" are used interchangeably to refer to a polymer of amino acid residues linked by amide bonds. As used herein, these terms apply to amino acid polymers in which one or more amino acid residues are artificial chemical analogues of a corresponding naturally occurring amino acid. Therefore, proteins may include natural and non-natural amino acids. Amino acids can be in the L or D form as long as the binding function of the peptide is maintained. Peptides may be cyclic, having an intramolecular bond between two non-adjacent amino acids within the peptide, for instance, backbone to backbone, side-chain to backbone and side-chain to side-chain cyclization. Cyclic peptides can be prepared by methods well know in the art; see e.g., US. Pat. No.6,013,625. Peptides can be of variable length, but are typically between about 4 and 200 amino acids in length.

The person skilled in the art knows various immune stimulatory polypeptides, transmembrane polypeptides, and tumor antigens that can be used for the present invention. The person skilled in the art can also generate fragments and derivatives (characterized by, e.g., substitution, deletion and/or addition of one or more amino acids), of such polypeptides and antigens which have substantially the same biological characteristics as the initial polypeptide, e.g., as regards immune stimulation, transmembrane anchoring, and tumor specificity according to routine methods.

Examples of immune stimulatory polypeptides that are suitable for the purposes of the present invention are GM-CSF, IL-2, IL-15, IL-18, MIP3β (CCL-19), MIP3a (CCL-20), CCL-21, CD40L, and NKG2D receptor ligands.

Preferably, said immune stimulatory polypeptide is a chemokine. Chemokines are small (7-16 kD), secreted, and structurally related soluble proteins that are involved in leukocyte and dendritic cell chemotaxis, PMN degranulation, and angiogenesis (Mackay, Nature Immunol. (2001) 2: 95-101; Gerard, et al., Nature Immunol. [2001] 2: 108-115). Chemokines are produced during the initial phase of host response to injury, allergens, antigens, or invading microorganisms. Chemokines selectively attract leukocytes to inflammatory foci, inducing both cell migration and activation. To function, chemokines are elaborated principally at the site of injury or infection and have a very short half-life due to their small size. Since systemic administration is associated with severe side effects (Giovarelli et al., J Immunol. [2000] 164: 3200-3206), it is difficult to obtain high local concentrations of chemokines in tumors in order to achieve an effective immune response.

The mechanism of chemokine action involves initial binding to specific transmembrane spanning G protein-linked receptors on target cells. The interaction of chemokines with such G protein-linked receptors causes a rapid reconfiguration of adhesion proteins, such as integrins, on the surface of the responding cells, facilitating their adhesion to endothelial cells lining blood vessel walls. This adhesion is followed by leukocyte transmigration between the endothelial cells into the tissues. Once there, the inflammatory leukocytes migrate along a gradient of increasing concentration of the chemokine to the site of origin. In response to the higher chemokine concentration at the site of injury or microbial invasion, the leukocytes are activated to perform effector functions such as release of their granule contents and increased production of cytokines.

Dendritic cells (DCs) are key initiators of innate and adaptive immunity. In addition to protection against pathogens, DCs also are thought to be central to the induction, regulation and maintenance of cellular immune responses against cancer cells (Gunzer et al., [2001] Sem Immunol 13:291-302; Timmerman and Levy, [1999] Annu Rev Med 50:507-529). DCs are potent antigen-presenting cells with the ability to stimulate primary immune responses and boost secondary immune responses. Immature DCs exit the bone marrow and enter the blood-stream and circulate to reach peripheric tissues where they remain in a physiological stage that is specialized for antigen capture, processing and presentation. Uptake of antigen by phagocytosis, macropinocytosis or receptor-mediated endocytosis, is followed by antigen processing and peptides thereof are presented on the cell surface associated with the MHC class I or II molecules. Then DCs receive maturation signals, either directly from pathogens or indirectly through inflammatory stimuli, which induce changes in the expression pattern of chemokine receptors, allowing DCs to migrate to draining regional lymphoid organs (Rescigno et al. [1999], Immunol Today 20:200-203). For instance, upregulation of CCR7 during maturation renders DCs reactive to CCL19 (also known as MIP-3β, ELC or exodus 3) and CCL21 (also known as 6Ckine, SLC or exodus 2), which direct their migration to T cell regions of regional lymphoid organs (Cyster [1999] Science 286:2098-2102; Salusto and Lanzavecchia [2000] Immunol Rev 177:134-140). There, DCs present immunogenic peptides in association with MHC class I and II molecules and induce activation and proliferation of specific CTLs and Th cells, respectively. Recently, CCL19 and CCL21 have been identified as potent maturation factors for DCs and indirect regulators of Th cell differentiation in mice. Thus, chemokines may not only organize the migration of DCs, but also are directly involved in the regulation of their ability to prime T cell responses.

The term "CCL19" used herein also comprises amino acid sequences that are substantially similar or include conservative variations of the human CCL19 protein sequence. The terms "substantially similar" and "conservative variation" denote the replacement of one or more amino acid residues by other biologically similar residues. Examples of conservative variations include the substitution of one polar residue for another, such as the substitution of arginine for lysine, glutamic acid for aspartic acid, or glutamine for asparagine or the substitution of one hydrophobic residue such as isoleucine, valine, leucine or methionine for another and the like. The terms "conservative variation" and "substantially similar" also include the use of a substituted amino acid in place of an unsubstituted parent amino acid provided that antibodies raised to the substituted polypeptide also are immunoreactive with the unsubstituted polypeptide.

Particularly preferred for the generation of the polypeptide of the present invention are the chemokines MIP-3β and IL-2. Examples of transmembrane polypeptides that are suitable for the purposes of the present invention are HBsAg, and Semliki fores virus (SFV) E2 envelope protein. Preferably, said transmembrane polypeptide is HBsAg(S).

Hepatitis B virus (HBV) is the prototypic member of the family Hepadnaviridae. HBV is an enveloped hepatotropic virus that causes chronic inflammatory liver disease in humans, which often leads to hepatocellular carcinoma. The HBV mature particle consists of a proteinaceous capsid made of subunits of the HBV core Ag (HBcAg) enclosing a circular incomplete double-stranded DNA genome, and an outer envelope made of the HBV surface antigen (HBsAg) and host lipids (Gilbert et al, 2005). There are three HBsAg forms: L (large), M (medium) and S (small), which share a common C-terminal domain and differ in their N-terminal amino acid sequences. In mature HBV particles about 80% of HBsAg is in the S form. HBsAg-S is a 226-amino acid hydrophobic protein multispanning transmembrane protein co-translationally translocated into the endoplasmic reticulum (ER) where it aggregates with nucleocapsids to form complete virus particles which bud into the lumen of the Golgi compartment and are secreted through the constitutive secretory pathway (Huovila et al., 1992; Bruss et al., 1991). Based on the Stirk model for the small S protein, HBsAg (S), the four transmembrane helices that are located between amino acid residues 8-28 (domain I), 79-100 (domain II), 160-184 (domain III) and 189-210 (domain IV) (Stirk et al., 1992; Berting et al., 1995; Sheu et al., 1994).

### Natural HBV VLPs

It is generally accepted that HBsAg-S spans the ER membrane four times and that both its N- and C-terminal domains and the hydrophilic antigenic "a" determinant face the ER lumen and therefore appear exposed on the surface of the mature virion (Prange et al., 1995; Clayton, 2001). In the process of HBV infection the infected hepatocytes release into the bloodstream large numbers of non-infective subviral particles devoid of nucleic acid, the so-called virus like particles (VLPs). These particles are composed of the major viral envelope protein, HBsAg-S, alone or associated with a small amount of the M subunit, and host-derived lipids (Hermann et al, 1984). Each spherical particle, with a diameter of 22 nm, contains about 100-120 HBsAg-S subunits stabilized by disulfide bonds (Ganem et al., 1987; Aggerbeck et al., 1985). The HBsAg-S VLPs are highly immunogenic and are used worldwide as vaccine to prevent infection by HBV as they trigger immune responses that neutralize the virus.

Expression of HBsAg-S in mammalian cells and yeast revealed that this protein has the capacity to self-assemble with host-derived lipids into VLPs without the requirement of nucleocapsids (Ganem, 1996; Laub et al., 1983; Liu et al., 1982). These VLPs are morphologically similar to the 22 nm plasma-derived particles of HBV infected individuals (Petre et al, 1987). This observation led to the development in the late 1980s of the first recombinant vaccine against HVB based on HBsAg-S particles produced in yeast (McAleer et al., 1984). Like the plasma-derived-particles, the recombinant HBsAg-S VLPs produced in yeast, insect and mammalian cells is a transmembrane polypeptide with the "a" determinant, a 60 aa domain containing major B-cell epitopes, and the amino and carboxyl termini facing the lumen of the ER and hence exposed on the surface of the VLPs (Eble et al., 1987; Stirk et al., 1992).

HBsAg-S offers important advantages over soluble and denatured proteins as immunogen. First, HBsAg-S VLPs are non-infectious and cannot replicate. In addition, the particulate nature of HBsAg-S VLPs seems to generate more effective immune responses. Vaccination with VLPs induces both B and T cell responses even in the absence of adjuvant (Schirmbeck et al., 1994; Gerlich et al., 1996; Netter et al., 2006). Further, HBsAg-S VLPs can be produced in large quantities in heterologous expression systems, and are easily enriched and purified ().

### Artificial Chimeric HBsAg-S

The capacity of HBsAg-S VLPs to elicit efficient immune responses has led to the construction of recombinant HBsAg-S containing foreign epitopes inserted close to the hydrophilic "a" determinant. This strategy has focused on maintaining VLP structure while providing effective presentation of the foreign antigen on the particle surface at predicted exposed domains close to the "a" determinant, around residues 59, 122-137 and 137-149 (Ionescu-Matiu et al., 1983). For instance, a poliovirus neutralizing (capsid) antigen (Delpeyroux et al., Science 233:472-475, 1986) were fused to HBsAg-S, aimed at generating effective poliovirus vaccine candidates, incorporated poliovirus sequences inserted at the hydrophilic regions within HBsAg-S (Delpeyroux et al., J. of Virology 1990 64(12):6090-6100). Another example is chimeric HBsAg-S VLPs made carrying Hepatitis C virus (HCV) antigens (Netter et al. 2001).

However, it has been reported that insertion of large peptides within the "a" determinant could result in VLP destabilization depending on the inserted sequence (Lee et al., J Med Virol, 1996, 50:145-51; Delpeyroux et al., J Mol Biol, 1987, 195: 343-350). N- and C-terminal fusions have been proposed as an alternative to incorporate larger peptides to the HBsAg-S VLPs that would also result in surface presentation. We have demonstrated previously that the fusion of human Papilloma virus (HPV) E7 protein, or a deletion mutant derivative E7Δ1-35, to the C-terminus of HBsAg-S does not interfere with proper assembly into VLPs and that such chimeric proteins are able to elicit specific antibody and T cell responses against E7 in mice (Baez-Astua et al., 2005; EP-B1 1243655).

Examples of tumor antigens that are suitable for the purposes of the present invention are gp100, MART-1/MElan A, gp75/TRP-1, tyrosinase, NY-ESO-1, melanoma proteoglycan, a MAGE antigen, a BAGE antigen, a GAGE antigen, a RAGE antigen, N-acetylglycosaminyltransferase-V, pi β-catenin, MUM-1, cyclin dependent kinase-4, p21-ras, BCR-abl, p53, p185 HER2/neu, epidermal growth factor receptor, carcinoembryonic antigen, modified cafc embryonic antigen, a carcinoma-associated mutated mucin, an Epstein Barr virus EBNA gene product, papilloma virus E7, papilloma virus E6, prostate specific antigen, prostate specific membrane antigen, KSA, kincsif 2, HIP-55, TGFβ-1 anti-apoptotic factor, tumor protein D52, HEFT, or an NY-BR antigen, or fragments or derivatives thereof having substantially the same tumor specificty. Particularly preferred tumor antigens are MAGE-1, MAGE-2, MAGE-3, MAGE-4, MAGE-6, MAGE-12, MAGE-51, GAGE-I, GAGE-2, RAGE-1, NY-BR-1, NY-BR-62,NY-BR-75, NY-BR-85, NY-BR-87, and NY-BR-96.

In a particularly preferred embodiment of the fusion polypeptide of the present invention, the tumor antigen comprises the amino acid sequence SCVYCKKEL, RCIICQRPL, SEYRHYQYS, ECVYCKQQL, TDLHCYEQL or RAHYNIVTF.

In a further preferred embodiment, the polypeptide of the present invention contains a Tag sequence, preferably at the C-terminus which might be useful for purification of the polypeptide, e.g., His-Tag, FLAG-Tag etc.. A preferred Tag sequence is a His-Tag. A particularly preferred His-Tag consists of 6 His-residues.

The various components (a), (b) etc. of the polypeptide of the present invention can be fused directly or can be separated by linker sequences, e.g., for avoiding steric hindrances. Suitable linker sequences are known to the person skilled in the art such as the amino acid sequence GGGGSGGGGS. For example, a linker might comprise 2 to 50 amino acid residues independently selected from the group consisting of glycine, serine, asparagine, threonine and alanine.

Preferably, the fusion polypeptide of the present invention has the following structure (from the N-terminus to the C-terminus):
(a)-(b)-(c);
(a₁)-(b)-(a₂)-(c);
(a)-(b)-(c)-Tag; or
(a₁)-(b)-(a₂)-(c)-Tag;
wherein a₁ and a₂ can be different or the same.

An example of fusion polypeptide of the present invention is a polypeptide which comprises:
(a) MIP-3β, or a fragment or derivative thereof having immune stimulatory activity;
(b) HBsAg(S), or a fragment or derivative thereof having the characteristics of a TM protein as regards membrane anchoring;
(c) IL-2, or a fragment or derivative thereof having immune stimulatory activity;
(d) a polytope comprising well-known CTL epitopes of the HPV16 E6 and/or E7 proteins, or fragments or derivatives thereof containing immunogenic amino acid sequences ; and, optionally
(e) a His-Tag.

Various examples of fusion polypeptides are given in Table 1.

**Table 1**

| FUSION POLYPEPTIDE | IL-2 ACTIVITY | MIP-3β ACTIVITY | CTL-RESP. | Ab-RESP. |
|---|---|---|---|---|
| MIP3β-p-HBsAg(S)-p-HPV16/18 E6E7 polytope-p-IL-2 | + | + | + | + |
| MIP3β-p-HBsAg(S)-p-IL-2-p-HPV16E6E7polytope | + | + | + | + |
| IL-2-p-HBsAg(S)-p-HPV16E6E7polytope-p-MIP3β | + | + | + | + |
| MIP3β-p-HBsAg(S)Δ⁹⁸-p-HPV16/18 E6E7 polytope-p-IL-2 | + | + | + | + |
| MIP3β-HBsAg(S)-BRCA-1 polytope-IL-2 | + | + | + | + |
| MIP3Δ-HBsAg(S)-IKBKEpolytope-IL-2 | + | + | + | + |
| MIP3β-HBsAg(S)-SURVIVIN polytope-IL-2 | + | + | + | + |

Abbreviations: BRCA-1, breast cancer 1 early onset; HBsAg, hepatitis B surface antigen; HPV16/18 E6E7, polytope of the E6 and E7 proteins of human papillomavirus types 16 and 18; IKBKE, I-kappa-B kinase e; IL-2, interleukin-2; MIP3β, macrophage inflammatory protein 3β; p, linker peptide

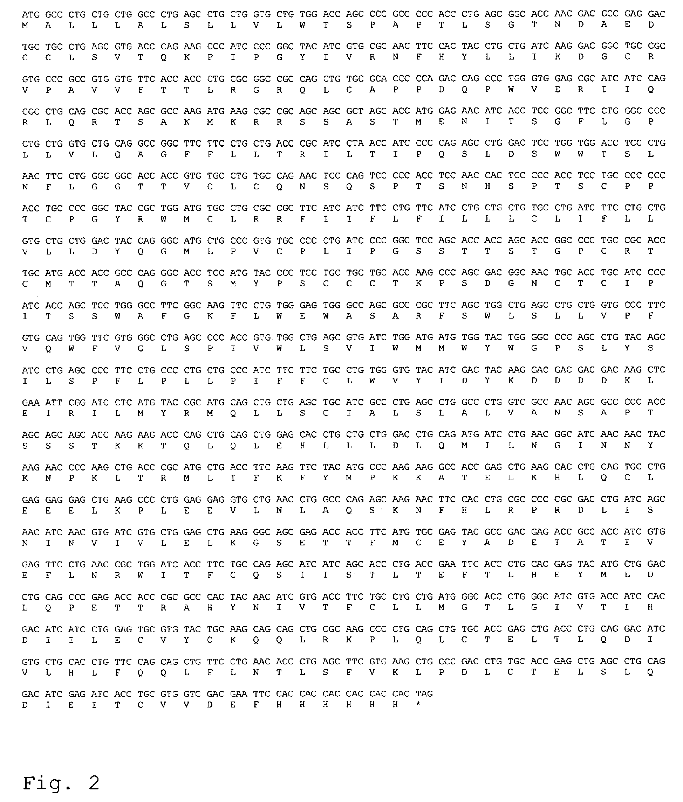

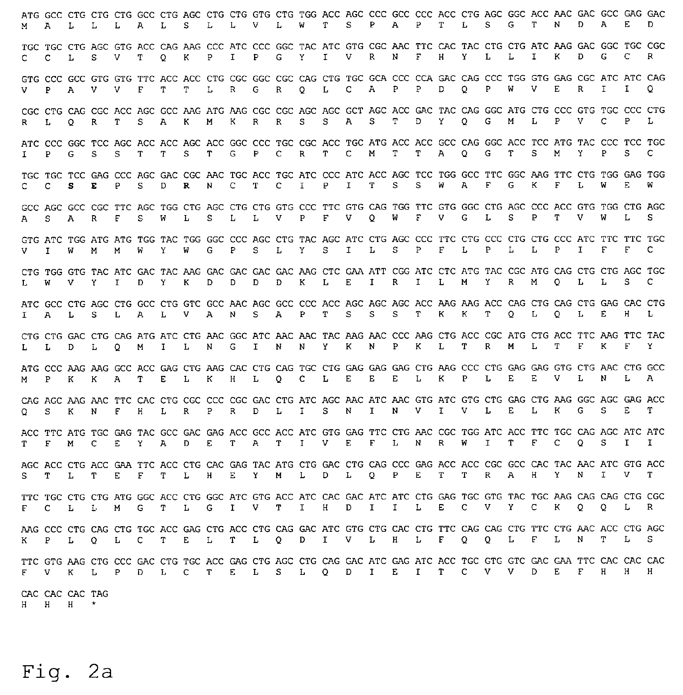

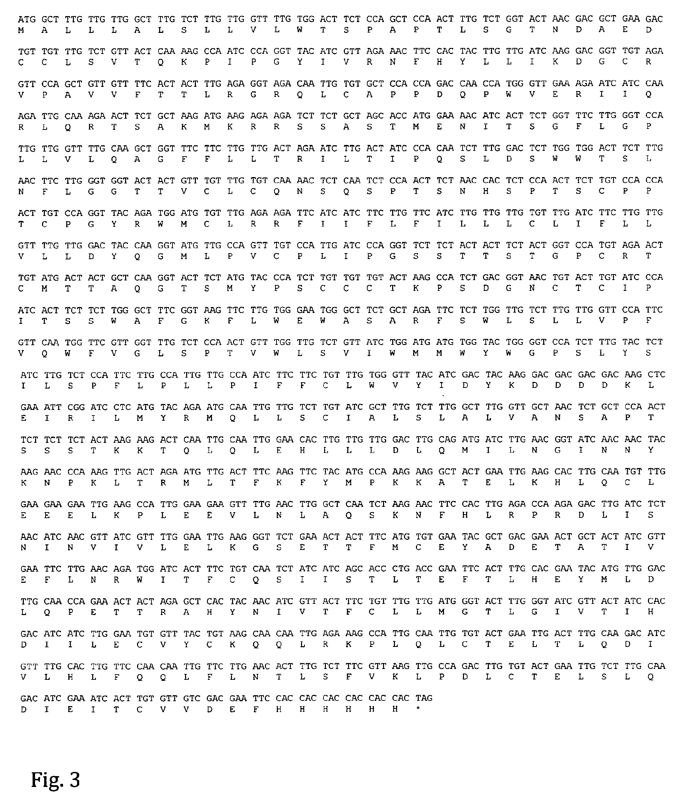

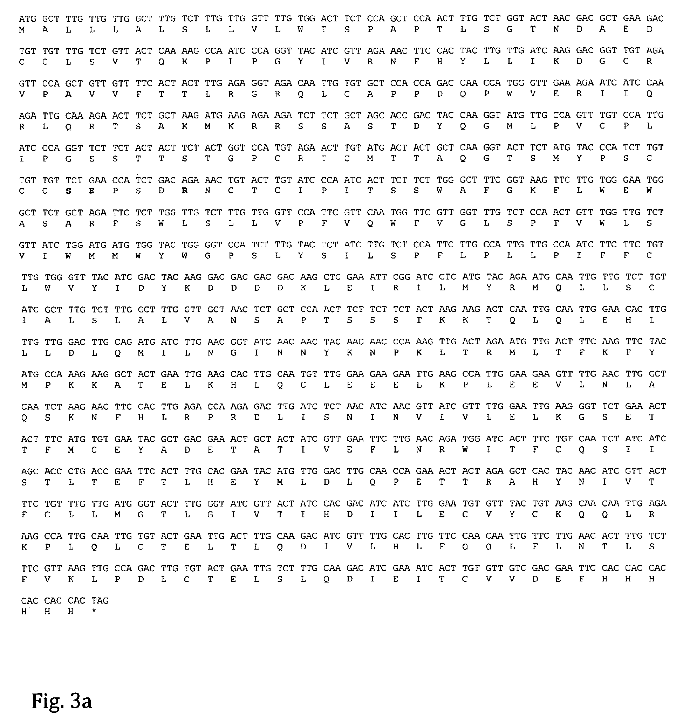

The present invention also relates to nucleic acid sequences encoding a fusion polypeptide of the invention. In a preferred embodiment, the nucleic acid sequence comprises the nucleic acid sequence shown in Figure 2(a) or Figure 3.

Moreover, the present invention also provides a recombinant vector containing a nucleic acid sequence of the present invention. The recombinant vectors can be constructed according to methods well known to the person skilled in the art; see, e.g., Sambrook, Molecular Cloning A Laboratory Manual, Cold Spring Harbor Laboratory [1992] N.Y. A variety of expression vectors, e.g., plasmids or viral vectors, may be utilised to contain and express nucleic acid sequences encoding a polypeptide of the present invention.

A preferred viral vector is a poxvirus, adenovirus, retrovirus, herpesvirus or adeno-associated virus (AAV). Particularly preferred poxviruses are a vaccinia virus, NYVAC, avipox virus, canarypox virus, ALVAC, ALVAC(2), fowlpox virus or TROVAC.

The present invention also provides a vaccine containing a polypeptide, nucleic acid sequence or vector of the present invention in an amount suitable for immunization of an individual and, preferably, one or more common auxiliary agents. The employed term "amount suitable for immunization of an individual" comprises any amount of a polypeptide, nucleic acid sequence or vector of the invention with which an individual can be immunized. The amount depends on whether immunization is intended as a prophylactic or therapeutic treatment. In addition, the individual's age, sex and weight play a role for determining the amount. Thus, the amount suitable for immunization of an individual refers to amounts of the active ingredients that are sufficient to affect the course and the severity of the disease, leading to the reduction or remission of such pathology. An ""amount suitable for immunization of an individual" may be determined using methods known to one skilled in the art (see for example, Fingl et al., The Pharmocological Basis of Therapeutics, Goodman and Gilman, eds. Macmillan Publishing Co., New York, pp. 1-46 [1975]). The term "individual" as used herein comprises an individual of any kind and being able to fall ill with carcinomas. Examples of such individuals are humans and animals as well as cells thereof.

The administration of the vaccine by injection may be made at various sites of the individual intramuscularly, subcutaneously, intradermally or in any other form of application. It may also be favourable to carry out one or more "booster injections" having about equal amounts.

The employed term "common auxiliary agents" comprises any auxiliary agents suitable for a vaccine to immunize an individual. Such auxiliary agents are, e.g., immunization adjuvants, such as GM-CSF or Freund's adjuvant, buffered common salt solutions, water, emulsions, such as oil/water emulsions, wetting agents, sterile solutions, etc.

A polypeptide, nucleic acid sequence or vector of the present invention can be present in the vaccine as such or in combination with carriers. It is favourable for the carriers in the individual not to be immunogenic. Such carriers may be the individual's own proteins or foreign proteins or fragments thereof. Carriers, such as serum albumin, fibrinogen or transferrin or a fragment thereof are preferred.

The immunogenic polypeptide, nucleic acid sequence or vector of the present invention can additionally be combined with an immunostimulatory adjuvant, e.g., alum salt, cholesterol, an immunostimulatory oligonucleotide, a saponin, tocopherol, oil-in-water emulsion, liposome, or a lipopolysaccharide.

The vaccine of the present invention may be therapeutic, that is, the compounds are administered to treat an existing cancer, or to prevent the recurrence of a cancer, or prophylactic, that is, the compounds are administered to prevent or delay the development of cancer. If the compositions are used therapeutically, they are administered to cancer patients and are designed to elicit an immune response to stabilize a tumor by preventing or slowing the growth of the existing cancer, to prevent the spread of a tumor or of metastases, to reduce the tumor size, to prevent the recurrence of treated cancer, or to eliminate cancer cells not killed by earlier treatments. A vaccine used as a prophylactic treatment is administered to individuals who do not have cancer, and are designed to elicit an immune response to target potential cancer cells or to target an antigen derived from a virus associated with cancer.

The present invention also relates to the use of a polypeptide, nucleic acid sequence or vector of the present invention for the production of a vaccine for the prevention or treatment of a carcinoma. For example, these may be a non-melanoma skin cancer, anogenital carcinoma (e.g., cancer of the uterine cervix (CxC) or a cervical intraepithelial neoplasia (CIN)), non-small cell lung carcinoma, bladder cancer or a head and neck neoplasia.

By means of the present invention it is possible to immunize individuals, in particular humans and animals. Immunization takes place by both induction of antibodies and stimulation of CD8⁺ T cells. Thus, it is possible to take prophylactic and therapeutic steps against carcinomas.

The below examples explain the invention in more detail.

### Example 1

### Materials and Methods

### (A) Animal model/species/strain/supplier

SPF healthy female C57BI/6 mice were obtained from Charles River. The animals were 6-12 weeks old at the beginning of experimentation. The animals were housed in Ventiracks (Tecniplas GmbH Deutschland, Hohenpeissenberg) in a single, exclusive room, air- conditioned to provide a minimum of 11 air changes per hour. The temperature and relative humidity ranges were within 20°C and 24°C and 40 to 70%, respectively. Lighting was controlled automatically to give a cycle of 12 hours of light and 12 hours of darkness. Specific pathogen free status was checked by regular control of sentinel animals. Diet: Throughout the study the animals had access ad libitum to sterilized diet type RM 1 (Harlan Laboratories Ltd., Itingen, Germany). Sterile water was provided ad libitum via bottles.

### (B) Cells characteristics/conditions of use

TC-1 tumor cells: These cells obtained from C57BI6 mice lung, have been transduced with 2 retroviruses: LXSN16E6E7 expressing E6 and E7 from HPV16 and pVEJB expressing the ras gene. TC-1 Cells were a kind gift of Dr TC Wu (The Johns Hopkins University, Baltimore, USA). The cells were cultured in DMEM containing 0.5 mg/ml G418 and 0.2 mg/ml Hygromycine. Adherents cells were removed by trypsine treatment and after 3 washings, tumor challenge were performed subcutaneously with 1x10⁵ TC-1 viable cells.

### (C) Protocol for Immunization

For the immunotherapeutic experiments, 15 C57BI6 female mice were challenged subcutaneously in the right flank with 1x10⁵ TC-1 cells at day 1. Mice were treated three times, subcutaneously at three distant sites, with MHIH on days 8, 15 and 22. MHIH was injected subcutaneously just before each immunization over the sites of injection to the shaved skin of mice (approx. 1 cm²). Tumor growth was monitored, twice a week during 80 days, with a calliper. Mice were sacrificed for ethical reasons when their tumor size was superior to 25 mm of diameter or when they showed pain even if the tumor was smaller.

For the immunogenicity study, 3 C57BI6 female mice were vaccinated subcutaneously at three distant sites with MHIH on days 1, 8, and 15. This dose was used to optimize the detection of cellular immunity against HPV specific antigens. 0.5 IU of tubertest, 5x10⁶ cfu of BCG, or 0.5% of levamisole was injected subcutaneously just before each immunization over the sites of injection.

### (D) Measure of the number/frequency of IFN-γ secreting cells by Elispot

Fresh spleen cells were prepared using Lympholite purification buffer. All the peptides were synthesized at DKFZ core facilities at the immunograde level (10 mg) . Each peptide was dissolved in DMSO at 10 mg/ml and stored at 4°C. A 96-well nitrocellulose plate was coated with 3 µg/ml monoclonal rat anti-mouse IFN-γ antibody (Clone R4-6A2; Pharmingen (Becton Dickinson GmbH, Heidelberg), cat. no. 551216, Lot M072862; 100 µl/well) in sodium carbonate buffer. The plates were incubated overnight at 4°C or 1 h at 37°C. Plates were washed three times with DMEM + 10% FCS and saturated 2 hours at 37 °C with 100 µl DMEM + 10% FCS/well. Splenocytes were plated at a concentration of 10⁶ cells/100 µl. Interleukine 2 (IL-2) was added to all the wells at a concentration of 6 U/50 µl/well (R&D Systems) 10 ng/ml). Concanavalin A was used as positive control (5 µg/ml). HPV specific peptides were used at a concentration of 5 µg/ml. The plates were incubated 48 hours at 37° C, 5% CO₂. The plate was washed one time with PBS 1X and 5 times with PBS-Tween 0.05%. Biotinylated anti-mouse IFN-γ (clone XMG 1.2, Pharmingen) was added at the concentration of 0.3 µg/100 µl/well and incubated 2 hours at room temperature under slow agitation. The plate was washed 5 times with PBS-Tween 0.05%. Extravidin AKP (Sigma, St. Louis, MO) diluted at 1/5000 in PBS-Tween 0.05%-FCS 1% was also added to the wells (100 µl/well). The plate was incubated 45 minutes at room temperature and then washed 5 times with PBS-Tween 0.05%. IFN-γ secretion was revealed with Biorad Kit. 100 µl substrate (NBT+BCIP) was added per well and plate was left at room temperature overnight. The plate was washed with water and put to dry overnight at room temperature. Spots were counted using a dissecting microscope.

### (E) T cell cultures

T cells were isolated from the spleens of immunized mice 7 days after the booster immunization. Spleen cell suspensions were filtered through 70 µm cell strainers (Beckton Dickinson) and pretreated for 5 minutes at room temperature with 0.8% NH₄Cl/0.1%KHCO₃ to deplete erythrocytes. After centrifugation, pellets of splenocytes were resuspended in RPMI 1640 supplemented with 10% FCS, 50 µM 2-mercaptoethanol, 10 I.U. of penicillin/ml, 20 µg of streptomycin/ml and 10 I.U./ml of rIL-2 (Roche, Mannheim) and incubated 2 hours at 37°C with 5% CO₂. Non-adherent cells were then collected and *in vitro* restimulated before performing the for ELISPOT analysis. T cells were restimulated *in vitro* with dendritic cells from naive mice loaded with E7⁴⁹⁻⁵⁷ peptide by addition to the culture medium (0.1 µM of peptide). Loaded cells were seeded at 2.5 x 10⁴ cells/well in RPMI 1640 supplemented with 5% FCS, 50 µM 2-mercaptoethanol and 10 IU/mL rIL-2 in 96-well plates. T cells were added to the wells of stimulator cells at 2 x 10⁵ cells/well.

### (F) CTL assay

Cytotoxic activity of T cells was assessed using a standard chromium release assay using the same effectors as in the ELISPOT assay. Target TC-1/A2 cells were labeled with 100 mCi 51Cr/10⁶ cells at 37°C for 2 hours and plated in each well of a 96-well, round-bottomed plate. T2 target cells pulsed with HPV-16 E7 peptide (amino acids 49-57) for 2 h at 37°C at the concentration of 1 mg/ml before chromium labeling, were used as positive control cells. After extensive washing, the labeled target cells were incubated with HLA-A2-restricted E7 (amino acids 49-57) peptide-stimulated T-cells. Peptide-stimulated T cells were produced by incubating 2 x 10⁶ Splenocytes from HLA-A2 (AAD) transgenic mice vaccinated with pIRES-neo2-MHIH plasmid were stimulated with irradiated TC-1/A2 cells in the presence of IL-2 for 5 days to generate effector cells. Negative controls included wells containing target cells but no effector cells (background). After a 4-h incubation, 100 µl of supernatant was harvested and ⁵¹Cr release was quantified using a gamma counter. Results are expressed as percent cytotoxicity ± SD calculated from the following formula: percent specific lysis_[specific release value - spontaneous release value) / (maximum release value-spontaneous release value)] x 100, where spontaneous release value represents counts in supernatants from wells containing just target cells, and maximum release value represents counts in supernatant from wells containing target cells in medium to which 10% SDS was added.

### (G) Electron microscopy

For the ultrastructural analysis of HBsAg VLPs, negative staining of purified fractions was performed. Briefly, 10 µl drops of each fraction were placed on Parafilm, grids were laid upside-down on top of the drops, incubated for 5 minutes at room temperature, washed in PBS and negative staining was performed with uranyl-acate (10 µl/grid, 1.5 minutes at room temperature). Samples were then rinsed in water, air dried and visualized and photographed with a Zeiss EM-10 microscope (Figure 5).

### (H) Western blotting

Samples of purified fractions were mixed with SDS loading buffer containing 1 mM DTT and boiled for 5 minutes at 95°C. The proteins were separated on 15% or 4-20% gradient gels (NuPAGE™, Invitrogen) polyacrylamide gels by SDS-PAGE, blotted onto PVDF membranes, blocked with 5% milk in PBS containing 0.1 % Tween 20 and incubated at room temperature with HRP-conjugated anti-FLAG M2 antibodies (Sigma). Antibody binding was visualized with enhanced chemiluminescence reagent (Renaissance®, NEN-Perkin Elmer) (Figure 6b).

### Example 2

### A vaccine to prevent and treat pre-cancer and cancer caused by high-risk human papillomaviruses

Human papillomavirus (HPV) is a genus of the papovaviridae family that comprises more than one hundred genotypes infecting skin or mucosal epithelia causing benign and malignant tumors. A subset of genital HPV, the so-called "high risk" HPV, are associated with anogenital carcinomas as well as head and neck neoplasias. Cancer of the uterine cervix (CxC) is the second leading cause of dead by cancer in women worldwide and the first in developing countries. About 500.000 new cases are diagnosed yearly with nearly 50% of global mortality. The most prevalent high-risk HPV types found in CxC are HPV16, which is associated with nearly 50% of cases and 18 which accounts for further 20%. In addition, a number of HPV types with tropism for the skin epithelium are associated with non-melanoma skin cancers. Both HPV types are also associated with head and neck cancers.

HPV infection of genital epithelia is very frequent, yet in most cases the HPV infection is asymptomatic and transient. However, epidemiological data indicate that HPV16 infection persists in approximately 10% of women for more than 2 years. These women are at high risk to develop high-grade pre-cancer lesions known as cervical intraepithelial neoplasia (CIN, grades I to III) that may progress to CxC.

The present example describes the generation of a safe therapeutic vaccine capable of stimulating the immune system in a way that results in prevention and treatment of HPV-associated cancers. To this end, a fusion gene named MHIH was constructed which encodes a polyprotein comprising the following parts: (A) the MIP-3β (CCL19) chemokine; (B) an HBsAg(S) moiety; (C) an IL-2 moiety; (D) an HPV polytope containing immunogenic sequences derived from the E6 and E7; and (E) a His-Tag. Two synthetic genes were constructed: (i) One was made of codons optimized for expression in human and mouse cells, i. e., 100% of codons were those preferred in highly expressed genes, as described in (Cid-Arregui et al., [2003) J Virol 77:4928-4937); and (ii) another synthetic gene with codons optimized for expression in the yeast S. cerevisiae (according to Gordon et al., [1986] Nuc Acids Res 14:5125-5143).

### List of tested peptides

SCVYCKKEL (E6; Db), S9L Peptide RCIICQRPL (E6; Db), R9L Peptide SEYRHYQYS (E6; Kb), S9S Peptide ECVYCKQQL (E6; Db), E9L Peptide TDLHCYEQL (E7; Kb), T9L Peptide RAHYNIVTF (E7; Db), R9F Peptide Irrelevant Peptide (e.g. influenza virus-specific).

### Results

### (A) Design and construction of synthetic genes

Codon optimization of genes for enhanced expression of the fusion proteins of this invention in human cells was performed by assembling synthetic sequences containing codons chosen among those preferred by highly expressed human genes, following an algorithm described previously (Cid-Arregui et al., J Virol. 77:4928-4937, 2003), as it was done for the complete HBsAg coding sequence (Genbank accession number AY515140) (Figure 2). Optimization of codons for enhanced expression in yeast, was carried out using an algorithm that was elaborated on the basis of the relative synonymous codon usage found in highly expressed genes from yeast as described in Sharp et al., Nucl. Acids Res. 14: 5125-5143 (1986 (Figure 3). The nucleotide sequences encoding the different parts of the fusion proteins were joined together in frame and the assembled genes were verified by sequencing. The superior expression driven by the synthetic genes was demonstrated in transfection experiments in which the wild type and codon-optimized genes were compared.

The synthetic genes were cloned into the plasmid pPICZA (Invitrogen) using the EcoRI and SalI restriction sites. The plasmids were purified and sequenced using flanking primers to confirm that the coding sequences were correct.

### (B) Cloning into the Picchia pastoris expression vectors

After assembly of the respective sequences, the fusion genes were cloned into the multiple cloning site of the plasmid pPICZA (Invitrogen) in frame with the sequence encoding the 6 x His-Tag contained in the plasmid just downstream the multiple cloning site.

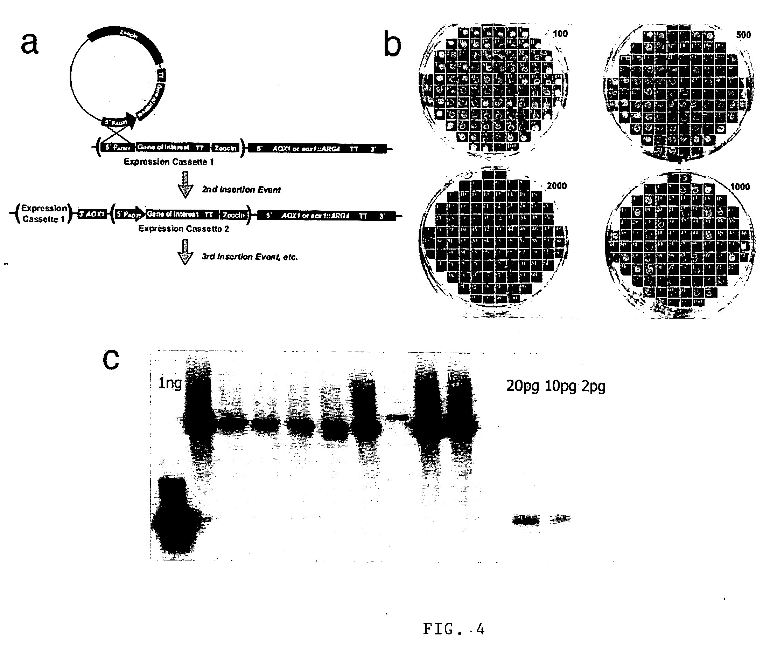

The pPICZA plasmids were linearized with PmeI and electroporated into P. pastoris competent cells (X-33 - strain). Transformed clones were selected in YPD plates containing 25 µg/ml of Zeocin. Subsequently, the clones were tested for resistance to increasing amounts of Zeocin up to 2000 µg/ml. Clones resistant to this antibiotic concentration were selected for further production of recombinant protein (Figure 4).

### (C) Production of recombinant fusion proteins using Picchia pastoris

The recombinant yeast *P. pastoris* (X-33 - strain) carrying pPICZ plasmids was cultivated in a synthetic medium to express fusion protein, based on the method reported previously in J. Biol. Chem. Vol. 267, No. 3, 1953-1961 (1992). Cultures set to initial 1 O.D. (A595 nm) in medium containing methanol as carbon source were inoculated from overnight pre-cultures (4-6 O.D. A595 nm) in medium with glycerol, and incubated at 29-30°C with orbital shaking for 6 days with daily addition of methanol (0.5%).

From the recombinant yeast in the stationary growth phase (after 5-6 days) the cells were collected by centriugation and whole cell extracts were prepared using Yeast Protein Extraction Reagent (Pierce Chemical Co.), then separated by SDS-PAGE, and subjected to silver staining to identify HBsAg in the sample. The recombinant yeast (wet weight: 20 g) cultivated on the synthetic medium 8S5N-P400 was suspended in 100 ml of lysis buffer (0.1 M sodium phosphate, pH 7.2, 15 mM EDTA, 2 mM PMSF, 0.1% Tween 20) and homogenized with a Bead-Beater using 0.5 mm glass beads. Then, the supernatant was recovered by centrifugation. Subsequently, the supernatant was mixed with 0.75-fold volume of 33% (w/w) PEG 6000, and cooled on ice for 30 minutes. Then, the mixture was centrifuged (7,000 rpm, 30 minutes) to recover pellets. The pellets were then re-suspended in lysis buffer without Tween 20.

The re-suspended pellets were layered over a CsCl solution of 10-40% gradient and subjected to ultracentrifugation at 28,000 rpm for 16 hours. After centrifugation, the sample was divided into 12 fractions, which were subjected to Western blotting (primary antibody was anti-HBsAg monoclonal antibody) to identify the fraction containing HBsAg. Then, the fractions containing HBsAg were dialyzed in lysis buffer without Tween 20.

The dialysate obtained previously (10 ml) was layered over a gradient of 5-50% sucrose and subjected to ultracentrifugation at 28,000 rpm for 16 hours. In the same manner as above, the fraction containing HBsAg fusion proteins after centrifugation was identified and dialyzed in lysis buffer containing 0.85% NaCl instead of urea and Tween 20.

The procedure was repeated and the sample after dialysis was concentrated using Ultra Filter Q2000 (Advantech Co.) and refrigerated at 4 degrees Celsius until use.

From the result of Western Blotting after CsCl density equilibrium centrifugation, The HE and MHIH fusion proteins were found to form particles by electron microscopy of negative staining of samples with uranyl acetate (Figure 5). These proteins have a molecular weight of ~30 and ~60 kDa, respectively (Figure 6). A total of about 15 mg of purified HBsAg particles were obtained from 20 g (wet weight) of the yeast mass derived from 2.5 L of culture medium.

The fractions obtained in the course of purification were analyzed by silver or coomassie staining after SDS-PAGE. Further, to confirm that protease derived from yeast was removed by purification, the purified particles obtained were incubated at 37°C for 12 hours, then subjected to SDS-PAGE, and identified by silver staining. Finally, it was confirmed that protease derived from yeast was completely removed by the overall purification process.

Protein extraction and purification by ion metal affinity chromatography. For affinity purification of recombinant protein by ion metal affinity chromatography (IMAC), supernatants from 2.3 were passed through Ni-NTA columns, washed three times with wash buffer and eluted in 1 ml fractions, which were kept at 4°C. The collected fractions were stored refrigerated until they were concentrated.

### (D) Concentration of purified proteins

After examination of the amount of protein in the different elution fractions and their reactivity with the specific antibodies, the elution fractions containing most of the recombinant protein were concentrated.

Concentration was carried out using Amicon Ultra 15 (Ultracel 10k) concentrators of 10 kDa pore (Millipore) by centrifugation at 3500 x g at 4°C in a swing bucket rotor. The particles were washed three times with 10 ml of either saline serum, isoosmolar buffer from Eppendorf or sterile Milli-Q water.

The particle suspensions were stored refrigerated until they were used for immunization experiments. The amount of protein in the different preparations and batches was determined using the BCA protein assay kit (Pierce).

### (E) Characterization of purified proteins

The eluted fractions were analyzed by SDS-PAGE, followed by Coomassie staining and Western blot detection with anti-HBsAg, anti-FLAG and anti-HIS and anti-CCL19 antibodies.

Antibodies used for detection:
- Anti-Hepatitis B surface antigen ad, ay (HRP-conjugated), rabbit polyclonal antibody. USBiological H1909-03.
- Anti-FLAG M2 mouse monoclonal antibody (HRP-conjugated), Sigma A8592.
- Anti-HIS tag antibody-HRP conjugate kit, Novagen 71840-3.
- Anti-MIP3 beta (CCL 19), USBiological M1202-66.

### (F) Electron microscopy

For the ultrastructural analysis of HBsAg VLPs, negative staining of purified fractions was performed. Briefly, 10 µl drops of each fraction were placed on Parafilm, grids were laid upside-down on top of the drops, incubated for 5 minutes at room temperature, washed in PBS and negative staining was performed with uranyl-acate (10 µl/grid, 1.5 minutes at room temperature). Samples were then rinsed in water, air dried and visualized and photographed with a Zeiss EM-10 microscope (Figure 5).

### (G) Western blotting

Samples of purified fractions were mixed with SDS loading buffer containing 1 mM DTT and boiled for 5 minutes at 95°C. The proteins were separated on 15% or 4-20% gradient gels (NuPAGE™, Invitrogen) polyacrylamide gels by SDS-PAGE, blotted onto PVDF membranes, blocked with 5% milk in PBS containing 0.1 % Tween 20 and incubated at room temperature with HRP-conjugated anti-FLAG M2 antibodies (Sigma). Antibody binding was visualized with enhanced chemiluminescence reagent (Renaissance®, NEN-Perkin Elmer) (Figure 6b).

### (H) Anti-tumor responses to vaccination

HLA-A2 transgenic mice have been extensively used to identify CTL epitopes that are recognized by the human immune system and to evaluate the efficacy of candidate therapeutic vaccines, since these mice may have CTL repertoires comparable to those found in humans. We have used HLA-A2.1(AAD) transgenic mice that express a chimeric HLA class I molecule, comprising the a-1 and a-2 domains from human HLA-A*0201 and the a-3 transmembrane/cytoplasmic domain from mouse H-2Dd (Ressing et al., 1995, J Immunol; 154: 5934-5943; Peng et al., 2006, Gene Therapy 13:67-77). The HLA-A2-restricted E7-specific CD8+ T-cell immune response induced by the MHIH vaccine was characterized tested in in HLA-A2 (AAD) transgenic mice. To this end, a syngeneic tumor cell line (TC1-A2) was used for a CTL assay to demonstrate specific killing of TC-1/A2 cells by E7 (amino acids 49-57)-specific CD8+ T cells (Figure 7). Splenocytes from HLA-A2 (AAD) transgenic mice vaccinated with pIRES-neo2-MHIH were isolatd and standard chromium release CTL assays were performed to assess the ability of E7⁴⁹⁻⁵⁷-specific T cells to lyse target cells.

### (I) IFN-γ ELISPOT assay

T cell responses were evaluated in HLA-A2 (AAD) transgenic mice by the IFN-γ ELISPOT assay. Groups of five HLA-A2 (AAD) transgenic mice were immunized three times at two-week intervals with 50 µg/dose of a eukaryotic expression plasmid (pIRES-neo2, Clontech) expressing either E7, HE or MHIH proteins. Six days after the third immunization the numbers of peptide specific, interferon gamma (IFN-γ)-producing cells were determined using Multiscreen-HA filtration plates (96-well, Millipore, Bedford, MA) that were coated overnight at 4°C with 5 µg/ml of anti-mouse IFN-γ antibody (clone R4-6A2, BD Pharmingen) in PBS. After washing with PBS, the plates were blocked with PBS/1% FCS for one hour at 37°C. Freshly isolated splenocytes were plated on pre-coated ELISPOT plates to 5x10⁴-1,25x10⁵ live cells per well in 100 µL culture medium (RPMI 1640, 10% FBS, 50 units/mL of penicillin/streptomycin) along with 10 IU/mL of IL-2 (Roche, Mannheim, Germany). The splenocytes were incubated for 24 h at 37 C/5% CO₂ in presence or absence of lµg/ml of HPV16 E7⁴⁹⁻⁵⁷ peptide. Then, the plates were washed twice with PBS/0.05% Tween 20, rinsed in PBS, and incubated overnight at 4°C with 2.5 µg/ml biotinylated anti-mouse IFN-γ antibody (clone XMG1.2, BD Pharmingen). Following extensive washing with PBS, the plates were incubated for 2 hours at room temperature with streptavidin-alkaline phosphatase conjugate (BD Pharmingen), diluted 1:500. After washing with PBS, the reaction was developed by addition of 5-bromo-4-chloro-3-3-indolyl phosphate/nitroblue tetrazolium solution (Sigma). Finally, the plates were washed with tap water and let dry overnight. The number of spots was measured with the aid of an Elispot reader (Zeiss-Vison C, Zeiss, Oberkochen, Germany). Figure 8 shows significantly stronger T cell responses to E7 in mice immunized with plasmid expressing MHIH fusion protein as compared with those immunized with plasmids expressing HBsAg-E7 or E7 alone.

### List of further references

Aggerbeck LP and Darrell LP, 1985; Virology 141:155-61.
Baez-Astua A et al., 2005, J Virol 79(20):12807-17.
Berting A et al., 1995, Intervirology, 38:8-15.
Bruss V et al., 1991 Proc Natl Acad Sci USA, 88: 1059-63.
Clayton RF, 2001 J Gen Virol, , 82: 1533-41.
Delpeyroux, F., N. Chenciner, A. Lim, Y. Malpie'ce, B. Blondel, R. Crainic, S. van der Werf, and R. E. Streeck. 1986. Science 233:472-475.
Delpeyroux F et al., 1987, J Mol Biol, 195: 343-350.
Eble BE et al, 1987, 1987, Mol Cell Biol, 7(10):3591-601 Ganem, D. 1996. Hepadnaviridae and their replication, p. 2703-2737. In B. N. Fields, D. M. Knipe, and P. M. Howley (ed.), Fields virology, 3rd ed. Lippincott-Raven Publishers, Philadelphia, Pa.
Ganem D et al., 1987; Annu Rev Biochem, 56:651-93.
Gao H. et al., 2005, PNAS 102(27):9469-74.
Gerlich, W. H., D. H. Krüger, and R. Ulrich (ed.). 1996, Intervirology 39:126-132.
Gilbert RJC et al., 2005, Proc Natl Acad Sci USA, 41: 14783-88 Gowans E.J et al., 2002.
Heermann et al., 1984, J Virol, 52:396-402.
Huovila AP et al., 1992,J Cell Biol 118: 1305-20.
Ionescu-Matiu et al., 1983, J Immunol, 130(4):1947-52.
Laub, O. et al., 1983, J Virol 48:271-280.
Lee IH et al., 1996,J Med Virol, 50:145-51
Liu et al, 1982, DNA 1:213-221.
McAleer WJ et al., 1984, Proc Soc Exp Biol Med, 175(3):314-9 Netter HJ et al. 2001, J. of Virology 75(5):2130-41.
Petre J, et al., 1987;Postgrad Med J. 63 Suppl 2:73-81.
Prange R et al., 1995, EMBO J, 14:247-256.
Schirmbeck, R., W. Boehm, and J. Reimann. 1996, Intervirology 39:111-119.
Sheu SY et al., 1994, J Gen Virol., 75:3031-9.
Stirk HJ et al., 1992; Intervirology. 33(3):148-58.

## Claims

1. A fusion polypeptide comprising in any order:
(1) at least one immune stimulatory polypeptide;
(2) at least one transmembrane (TM) polypeptide; and
(3) at least one tumor antigen; or
(4) a biologically active derivative or fragment of (1), (2), and/or (3) .

2. The fusion polypeptide of claim 1, wherein the immune stimulatory polypeptide is a chemokine.

3. The fusion polypeptide of claim 2, wherein the chemokine is MIP-3β and/or IL-2.

4. The fusion polypeptide of any one of claims 1 to 3, wherein the TM polypeptide is a self-assembling carrier molecule.

5. The fusion polypeptide of claim 4, wherein the carrier molecule is HBsAg(S).

6. The fusion polypeptide of claim 5, wherein the HBsAg(S) polypeptide is a deletion mutant (a) HBsAgΔ98 having an amino-terminal deletion of the first 98 amino acids of the HBsAg (S) protein of 226 amino acids or (b) HBsAgΔ153 having an amino-terminal deletion of the first 153 amino acids of the HBsAg(S) protein of 226 amino acids.

7. The fusion polypeptide of any one of claims 1 to 6, wherein the tumor antigen is gp100, MART-1/MElan A, gp75/TRP-1, tyrosinase, NY-ESO-1, melanoma proteoglycan, a MAGE antigen, Tyr1 and Tyr2, c-Met, PSA, PSM, thyroperoxidase, GaINAc, p-HCG, p97, alpha-fetoprotein, a BAGE antigen, a GAGE antigen, a RAGE antigen, N-acetylglycosaminyltransferase-V, pi β-catenin, MUM-1, cyclin dependent kinase-4, p21-ras, BCR-abl, p53, p185 HER2/neu, epidermal growth factor receptor, carcinoembryonic antigen, modified cafc embryonic antigen, a carcinoma-associated mutated mucin, a member of the pMel 17 gene family, an Epstein Barr virus EBNA gene product, papilloma virus E7, papilloma virus E6, prostate specific antigen, prostate specific membrane antigen, KSA, kincsif 2, HIP-55, TGFß-1 anti-apoptotic factor, tumor protein D52, HEFT, or an NY-BR antigen, or a fragment or derivative thereof.

8. The fusion polypeptide of claim 7, wherein the tumor antigen is MUC1, MUC2, MUC3, MUC4, MUC18, CEA, MAGE-1, MAGE-2, MAGE-3, MAGE-4, MAGE-6, MAGE-12, MAGE-51, GAGE-I, GAGE-2, RAGE-1, NY-BR-1, NY-BR-62,NY-BR-75, NY-BR-85, NY-BR-87, or NY-BR-96.

9. The fusion polypeptide of claim 7 or 8, wherein the tumor antigen is a mutant derivative from those naturally occurring in cancer cells.

10. The fusion polypeptide of any one of claims 1 to 9, further containing a Tag sequence.

11. The fusion polypeptide of claim 10, wherein the Tag sequence is a His-Tag.

12. The fusion polypeptide of any one of claims 1 to 11, wherein the tumor antigen comprises the amino acid sequence SCVYCKKEL, RCIICQRPL, SEYRHYQYS, ECVYCKQQL, TDLHCYEQL or RAHYNIVTF.

13. The fusion polypeptide of any one of claims 1 to 12 having the following structure from the N-terminus to the C-terminus: (A)-(B)-(C);
(A)-(B)-(A)-(C):
(A)-(B)-(C)-TAG; or
(A)-(B)-(A)-(C)-TAG;

14. The fusion polypeptide of any one of claims 1 to 13, comprising
(a) MIP-3β (CCL19), or a fragment or derivative thereof having immune stimulatory activity;
(b) HBsAg(S), or a fragment or derivative thereof having the characteristics of a TM protein;
(c) IL-2, or a fragment or derivative thereof having immune stimulatory activity;
(d) E6 and E7, or fragments or derivatives thereof containing immunogenic amino acid sequences; and, optionally,
(e) a His-Tag.

15. The fusion polypeptide of claim 14 comprising the amino acid sequence depicted in Figure 2.

16. A nucleic acid sequence encoding the fusion polypeptide of any one of claims 1 to 15.

17. The nucleic acid sequence of claim 16 comprising the nucleic acid sequence shown in Figure 2(a) or Figure 3.

18. A vector containing the nucleic acid sequence of claim 16 or 17.

19. The vector of claim 18, which is a plasmid or viral vector.

20. The vector of claim 19, wherein the viral vector is a poxvirus, adenovirus, retrovirus, herpesvirus or adeno-associated virus (AAV).

21. The vector of claim 20, wherein the poxvirus is a vaccinia virus, NYVAC, avipox virus, canarypox virus, ALVAC, ALVAC(2), fowlpox virus or TROVAC.

22. A vaccine containing a fusion polypeptide of any one of claims 1 to 15, a nucleic acid sequence of claim 16 or 17, or a vector of any one of claims 18 to 21.

23. A fusion polypeptide of any one of claims 1 to 15, a nucleic acid sequence of claim 16 or 17, or a vector of any one of claims 18 to 21 for use in a method for the prevention or treatment of cancer.

24. Use of a fusion polypeptide of any one of claims 1 to 12, a nucleic acid sequence of claim 13 or 14, or a vector of any one of claims 18 to 21 for the preparation of a vaccine for the prevention or treatment of cancer.

25. The use of claim 24, wherein said cancer is non-melanoma skin cancer, non-small cell lung carcinoma, bladder cancer, anogenital carcinoma or a head and neck neoplasia.

26. The use of claim 25, wherein said anogenital cancer is a cancer of the uterine cervix (CxC) or a cervical intraepithelial neoplasia (CIN).

27. A method for generating a fusion polypeptide of any one of claims 1 to 6 comprising the identification of one or more tumor-specific antigens or mutant forms in a cancer patient and incorporation of such antigen into the fusion polypeptide.

28. A method of transducing cells from a cancer patient with a vector of any one of claims 18 to 21 comprising the isolation of the cells from the patient and transduction of said cells ex vivo.
